# EUROPEAN PATENT SPECIFICATION

(11) **EP 0 922 435 B1**
(45) Date of publication and mention of the grant of the patent: **17.10.2007**
(21) Application number: 98911145.5
(22) Date of filing: 31.03.1998
(51) Int. Cl.: A61B 17/03, A61B 17/072

(54) **SUTURE RETAINING MEMBER FOR USE IN MEDICAL TREATMENT**
NÄHFADENHALTEVORRICHTUNG FÜR ÄRZTLICHE BEHANDLUNG
ELEMENT DE MAINTIEN DE SUTURE UTILISABLE A DES FINS MEDICALES

(30) Priority: 31.03.1997 JP 7868197
(43) Date of publication of application: 16.06.1999
(73) Proprietor: KABUSHIKIKAISHA IGAKI IRYO SEKKEI, Kyoto-shi, Kyoto 607-8035 (JP)
(72) Inventor: IGAKI, Keiji, Shiga 525-0045 (JP)
(74) Representative: Hoffmann, Jörg Peter
(86) International application number: PCT/JP1998/001490
(87) International publication number: WO 1998/043543

(56) References cited:
- EP-A- 0 636 378
- FR-A- 2 736 817
- JP-A- 9 024 050
- US-A- 4 834 090
- US-A- 5 542 594
- PATENT ABSTRACTS OF JAPAN vol. 1997, no. 05, 30 May 1997 (1997-05-30) & JP 09 024050 A (IGAKI IRYO SEKKEI:KK;GUNZE LTD), 28 January 1997 (1997-01-28) & EP 0 770 357 A 2 May 1997 (1997-05-02)
- PATENT ABSTRACTS OF JAPAN vol. 1996, no. 06, 28 June 1996 (1996-06-28) & JP 08 047526 A (GUNZE LTD), 20 February 1996 (1996-02-20)

## Description

### Technical Field

This invention relates to a medical suture holding member sewed to a site of abscission of a living tissue. More particularly, it relates to a medical suture holding member loaded in use on an automatic sewing device.

### Background Art

Abscission of a nidus formed in a living tissue is usually made by surgical operation. For reducing the load on a patient, operations under observation through an endoscope have come into widespread use.

An example is abscission employing an automatic sewing device. The automatic sewing device is introduced into a living body via a perforation to resecte the lesion and to sew the resected site together, and has an advantage that incision of the lesion is unnecessary by its use. Thus, the automatic sewing device has a sewing unit at its distal end for clinching the site of abscission of the living tissue. This sewing unit includes a plurality of staple lines for suture and a cutter for resecting the living tissue.

Of course, the use of the automatic sewing device is useful in the surgical operations for shortening the suture or incision time or simplifying the operation.

Meanwhile, in doing the operations by the above automatic sewing device for a fragile tissue, such as the tissue of the lung, bronchus, liver or the digestive tract, suture solely by a staple tends to lead to breakage of the tissue. In the case of operations for the lung, air leakage tends to be produced. This air leakage is fatal to the patient so that positive prevention of air leakage is a strongly desired.

It has thus been practiced to load a suture holding member on the automatic sewing device and to sew this suture holding member by a staple to the site of abscission of the living tissue.

For enabling loading of the suture holding member on the automatic sewing device and for allowing it to reach the site of lesion reliably, some means need to be used. For example, it has been proposed to apply an extensible nit fabric on a felt-like suture holding member and to sew them together along both side edges to a tubular shape.

However, with the suture holding member obtained on sewing an extensible nit fabric by a sewing yarn to enable it to be loaded on the automatic sewing device, the suture holding member tends to be contracted in the inserting direction and to be offset or rotated (twisted) towards the proximal end of the automatic sewing device around the automatic sewing device.

Such offsetting or rotation of the suture holding member cannot be coped with effectively, so that it presents a serious problem on the occasion of surgical operations under a limited field of view, such as under observation through an endoscope.

FR-A-2 736 817 discloses a medical suture holding member loaded in use on an automatic sewing device having a staple line that comprises main body portion of a holding member formed into a tubular shape by extrusion of a natural or synthetic high molecular material and having an opening on its proximal end for enabling loading of the main body portion of the holding member on a sewing unit of the automatic sewing device. Such member may be provided with a detachment preventative means for preventing detachment of the member from the automatic sewing device. Such detachment preventative means is formed as a thickened portion of the medical suture holding member.

Patent Abstracts of Japan vol. 1997, no. 05, 30 May 1997 and JP 09 024050 A disclose a suture material for medical treatment which has a closed distal end.

### Disclosure of the Invention

It is therefore an object of the present invention to provide a novel medical suture holding member capable of overcoming the problem inherent in the above-described conventional suture holding member.

It is another object of the present invention to provide a medical suture holding member which can be easily inserted into an automatic sewing device and which is not subjected to inconveniences due to offsetting or rotation at the time of insertion into the automatic sewing device to enable positive prevention of air leakage when the suture holding member is used in surgical operations for a lung.

It is still another object of the present invention to provide a medical suture holding member which can be easily inserted into an automatic sewing device and which enables its positive loading on the automatic sewing device.

It is yet another object of the present invention to provide a medical suture holding member which can be smoothly introduced into the automatic sewing device.

For accomplishing the above objects, the present invention provides a medical suture holding member loaded in use on an automatic sewing device having a staple line, including a main body portion of a holding member molded integrally to a tubular shape from a natural or synthetic high molecular material and having an opening on its proximal end for enabling loading of the main body portion of the holding member on a sewing unit of the automatic sewing device, according to claim 1.

If the present suture holding member is molded to a tube having a closed distal end, it is possible to prevent offsetting otherwise produced due to contraction of the suture holding member in the inserting direction thereof at the time of insertion into the automatic sewing device.

Moreover, with an integrally molded structure of the medical suture holding member, having a closed distal end, the loading portion has its entire periphery covered by a suture holding member when the medical suture holding member is loaded on the automatic sewing device, so that the suture holding member can be reliably positioned at a sewing site in the living body.

The medical suture holding member of the present invention has detachment preventative means in a main body portion of the holding member to prevent possible detachment of the suture holding member from the automatic sewing device on loading the suture holding member on the automatic sewing device. As means for preventing such detachment, the proximal end of the main body portion of the holding member formed with the opening is formed with a thickened portion for increasing elasticity thereat as compared to other portions. Alternatively, for preventing detachment of the suture holding member form the automatic sewing device, the inner peripheral surface of the main body portion of the holding member is a roughed surface presenting micro-sized irregularities. Still alternatively, plural ribs are formed on the inner peripheral surface of the main body portion of the holding member.

For facilitated confirmation of the loading state on the automatic sewing device, a display line extending axially of the main body portion of the holding member is provided on the main body portion of the holding member.

For aiding in grasping the quantity of the tissue of the living body, held by the automatic sewing device, a graduated line having fixed graduation widths is provided on the main body portion of the holding member.

The processing aimed at providing a hydrophilic surface is performed to assure facilitated loading in the living body.

If the suture holding member is made up of a pair of main body portions of the holding member, one of which is provided on a sewing unit carrying a knife blade or a cartridge holding plural staples of the automatic sewing device and the other of which is loaded on a stapling die adapted to hold the living tissue with the sewing unit to receive the staples, the respective main body portions are different in color to assure facilitated distinction between the main body portions provided on the rear portion of the sewing device and on the stapling die.

As the material for the medical suture holding member, it is possible to use a high molecular material that can be molded to a tube having a closed distal end. The high molecular material is preferably such a material that can be reliably loaded on the automatic sewing device and that is extendable and flaccid at room temperature to facilitate loading.

Specifically, the high molecular material is a natural high molecular material such as natural rubber, collagen, gelatine, chitin-chitosan, or a synthetic material, such as aliphatic polyesters, fatty acid anhydrides, aliphatic polycarbonates, polyphosphasen or a copolymer containing at least one of them.

In particular, for facilitating post-operation processing, it is desirable to use a biologically absorptive high polymer material, such as polyglycolicacid, polylactic acid, poly-ε-caprolactone, poly-β-hydroxy alkanoate or copolymer containing at least one of them.

By providing a weakened line, the main body portion of the holding member can be separated and taken out easily.

### Brief Description of the Drawings

Fig.1 is a perspective view showing a medical suture holding member according to the present invention.
Fig.2 is a cross-sectional view showing the medical suture holding member shown in Fig. 1.
Fig.3 is a cross-sectional view showing another embodiment of the medical suture holding member according to the present invention.
Fig.4 is a cross-sectional view showing a further embodiment of the medical suture holding member according to the present invention.
Fig.5 is a cross-sectional view showing a still further embodiment of the medical suture holding member according to the present invention.
Fig.6 is a cross-sectional view showing still another embodiment of the medical suture holding member according to the present invention.
Fig.7 is a cross-sectional view showing yet another embodiment of the medical suture holding member according to the present invention.
Fig.8 is a schematic perspective view showing an embodiment of an automatic sewing device employing the suture holding member according to the present invention.
Fig.9 is a schematic perspective view showing essential parts of a sewing unit of the automatic sewing device.
Fig.10 is schematic perspective view showing the state of loading of the suture holding member on the automatic sewing device.
Fig.11 is a schematic perspective view showing the sewing state by the automatic sewing device.
Fig.12 is a schematic perspective view showing the state of the end of the sewing operation.
Fig.13 is a perspective view of a medical suture holding member having a display line on a main body portion on a holding member according to the present invention.
Fig.14 is a perspective view of a medical suture holding member having a graduated line on a holding member according to the present invention.
Fig.15 is a perspective view showing a further embodiment of the medical suture holding member according to the present invention.
Fig.16 is a perspective view showing a still further embodiment of the medical suture holding member according to the present invention.
Fig.17 is a perspective view showing a further example of a main body portion of a holding member constituting a medical suture holding member according to the present invention.
Fig. 18 is a perspective view of a medical suture holding member having an insertion smoothing layer formed on the surface of the main body portion of the holding member shown in Fig. 16.
Fig.19 is a perspective view showing an embodiment in which a weakened line for cutting is provided in the main body portion of the holding member of the medical suture holding member.
Fig.20 is a perspective view showing a further embodiment of the medical suture holding member in which a weakened line for cutting is provided in the main body portion of the holding member.
Fig.21 is a perspective view showing the state in which the distal end of the main body portion of the holding member has been separated along the weakened line for cutting.
Fig.22 is a perspective view showing a still further embodiment of the medical suture holding member in which a weakened line for cutting is provided on the main body portion of the holding member.
Fig.23 is a perspective view showing another embodiment of the medical suture holding member in which a weakened line for cutting is provided on the main body portion of the holding member.

### Best Mode For Carrying Out the Invention

Referring to the drawings, preferred embodiments of a medical suture holding member according to the present invention will be explained in detail.

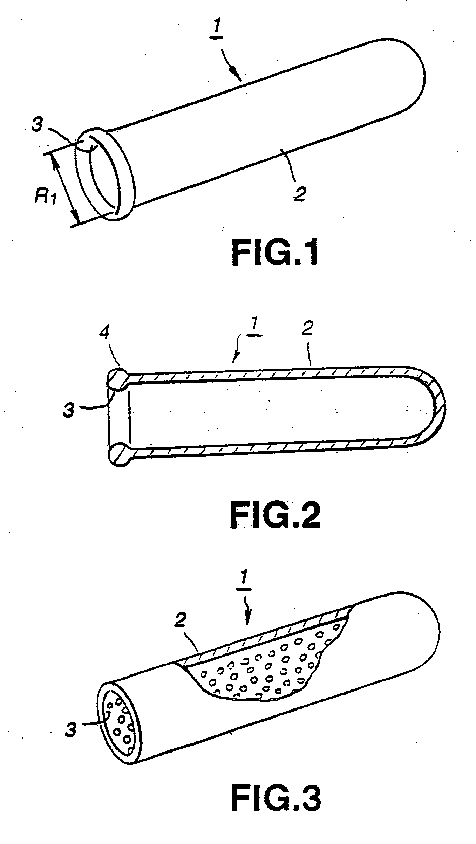

A medical suture holding member 1 includes a main body portion of a holding member 2 as shown in Fig. 1. The main body portion of a holding member 2 includes an opening 3 at its proximal portion. The main body portion of a holding member 2 serves for mounting a staple supporting die for receiving a sewing unit constituting a sewing portion of an automatic sewing device or a staple protruded therefrom to warp the staple. That is, the medical suture holding member 1 is loaded on the outer rim of the staple supporting block or the sewing unit via the opening 3.

The distal end of the main body portion of a holding member 2 is closed. That is, the main body portion of a holding member 2 is molded integrally to a tube closed at its distal end and which has an opening 3 at its proximal end.

This main body portion of a holding member 2 can be molded by injection molding a high molecular material using a metal mold for injection molding. Alternatively, a high molecular material is injection-molded to form a preliminary molded tubular article which is the molded by blow-molding.

The main body portion of a holding member 2 is formed to a length substantially equal to the length of the loading portion of the automatic sewing device as will be explained subsequently.

As a material making up the main body portion of a holding member 2, a natural or synthetic high-molecular material is used. As the high-molecular material, such a material which exhibits flexibility and flaccidness at ambient temperature of approximately 10° to 30° is used. The purpose of using this material is to load reliably the tubular main body portion of a holding member 2 closed at its distal end on an automatic sewing device and to facilitate the loading. If the main body portion of a holding member 2 is molded from a material flexible and flaccid at room temperature, the diameter of the inner rim is set so as to be slightly smaller than the outer diameter of the loading unit of the automatic sewing device so that the main body portion of a holding member 2 is loaded on the loading unit of the automatic sewing device by exploiting the elasticity proper to the body portion of a holding member 2.

The material of the main body portion of a holding member 2 may be enumerated by natural high-molecular material, such as natural rubber, collagen, gelatine, chitin or chitosan.

As the natural high-molecular material, a protective film for an organ, such as a pericardial film may be used.

The material constituting the main body portion of a holding member 2 may be enumerated by aliphatic polyesters, fatty acid anhydrides, aliphatic polycarbonates, polyphosphasen or copolymers containing at least one of these materials, and high-molecular materials exhibiting biological absorptivity, such as polyglycolic acid, polylactic acid, poly-ε-caprolactone, poly-β-hydroxy alkanoate, or copolymers containing at least one of these materials.

If poly-ε-caprolactone, as a bioabsorptive synthetic high-molecular material, is used, a copolymer thereof with other high-molecular materials, containing at least 30 mol% of poly-ε-caprolactone, may be used.

If poly-β-hydroxy alkanoate, as a bioabsorptive synthetic high-molecular material, is used, a copolymer thereof with other high-molecular materials, containing at least 30 mol% of poly-β-hydroxy alkanoate, may be used.

The suture holding member 1 may also be molded from a complex material composed of the above-mentioned natural high-molecular material and the synthetic high-molecular material.

As the material constituting the main body portion of a holding member 2, bioabsorptive natural and synthetic high-molecular materials are preferably used. By using the bioabsorptive materials, it is possible to eliminate the operation of removing the suture holding member 1 after the surgical operations.

Meanwhile, the suture holding member 1 according to the present invention includes a mechanism for preventing detachment from the automatic sewing device when the suture holding member is loaded on the automatic sewing device.

The proximal end of the main body portion of a holding member 2 formed with the opening 3 has a thickened portion 4 thicker than other portions. This thickened portion 4 is molded integrally with the main body portion of a holding member 2 and is molded simultaneously with the holding member 2.

The thickened portion 4 is more elastic than the remaining portions when the main body portion of a holding member 2 is molded from the natural or synthetic high-molecular material exhibiting elasticity. When the suture holding member 1 is loaded on the automatic sewing device, a larger pressure bonding force can be developed by this thickened portion 4. Thus, the suture holding member 1 may be prevented from inadvertent detachment from the automatic sewing device.

It suffices if the thickened portion 4 has the proximal end of the main body portion of a holding member 2 thicker than the remaining portions. The thickened portion 4 may be swollen in its shape to the inner or outer rim side of the main body portion of a holding member 2.

For preventing inadvertent detachment of the suture holding member 1 from the automatic sewing device, the inner peripheral surface of the main body portion of a holding member 2 is a roughed surface having micro-sized irregularities 5. By roughing the inner peripheral surface of the main body portion of a holding member 2, it is possible to enlarge the force of friction between the suture holding member 1 and the automatic sewing device to prevent inadvertent detachment of the suture holding member 1 from the automatic sewing device when the suture holding member 1 is loaded on the automatic sewing device.

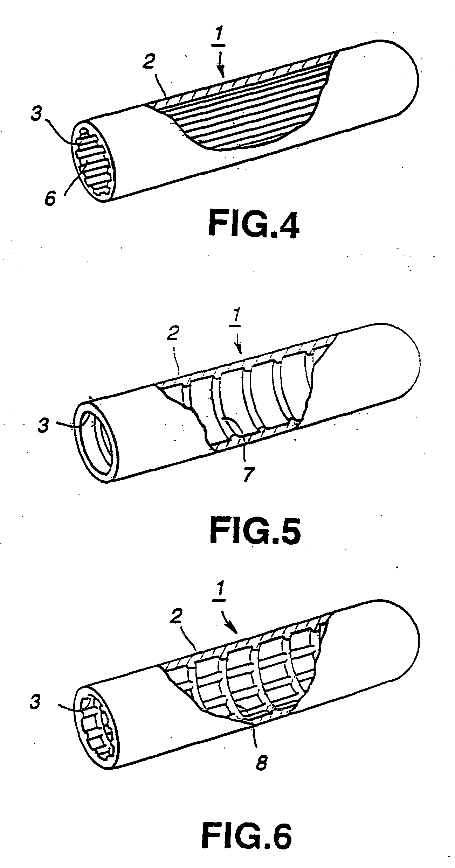

On the inner peripheral surface of the main body portion of a holding member 4 are formed plural ribs 6 substantially parallel to one another in an axial direction from the proximal end provided with the opening 3 towards the distal end, as shown in Fig.4. By providing the ribs 6, the inner peripheral surface of the main body portion of a holding member 4 is roughed to increase the force of friction between the suture holding member 1 and the automatic sewing device to prevent inadvertent detachment of the suture holding member 1 from the automatic sewing device.

It is also possible to provide the inner peripheral surface of the main body portion of a holding member 4 with plural ribs 7 around the periphery of the main body portion of a holding member 4 at a fixed distance from and substantially parallel to one another, as shown in Fig.5. In this case, the inner peripheral surface of the main body portion of a holding member 4 is roughed to increase the force of friction between the suture holding member 1 and the automatic sewing device to prevent inadvertent detachment of the suture holding member 1 from the automatic sewing device.

On the inner peripheral surface of the main body portion of a holding member 4, mesh-like ribs 8 may be formed, as shown in Fig.6. In such case, the inner inner peripheral surface of the main body portion of a holding member 4 is roughed to provide an increased force of friction between the suture holding member 1 and the automatic sewing device to prevent inadvertent detachment of the suture holding member 1 from the automatic sewing device.

In addition to forming micro-sized irregularities 5 or ribs 6 to 8 on the inner peripheral surface of the main body portion of a holding member 4, as shown in Figs.3 to 6, a thickened portion 4 may be provided on the proximal side of the main body portion of a holding member 2, as shown in Fig.2. By further forming the thickened portion 4 on the main body portion of a holding member 2, the suture holding member 1 can be prevented more reliably from being detached from the automatic sewing device.

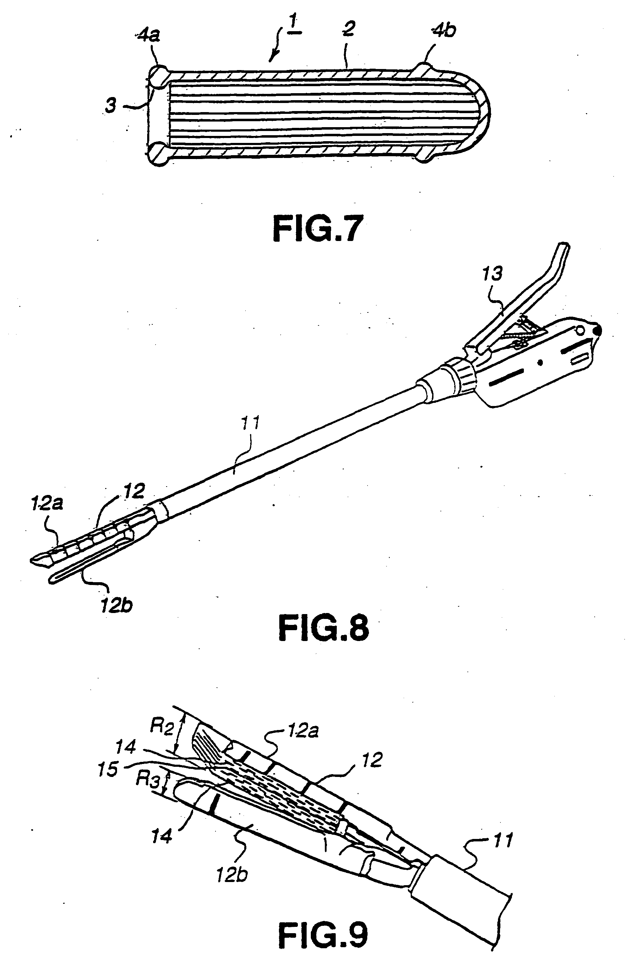

If the ribs are provided substantially parallel to one another on the inner peripheral surface of the main body portion of a holding member 4, as shown in Fig.4, thickened portions 4a, 4b are formed in the circumferential direction of the main body portion of a holding member 2 at the proximal end carrying the opening 3 and on the closed distal end of the main body portion of a holding member 2, as shown in Fig.7. By providing the thickened portions 4a, 4b at the proximal and distal ends of the main body portion of a holding member 2, the suture holding member 1 can be loaded on the automatic sewing device without twisting the ribs 6 when the suture holding member 1 is mounted on the automatic sewing device.

The above-described suture holding member is loaded in use on the automatic sewing device. The structure of the automatic sewing device and the method of use of the suture holding member 1 are hereinafter explained.

The automatic sewing device has an openable sewing unit 12 at a distal end of a shaft 11 and carries an operating unit 13 for holding and actuation, as shown in Fig. 8.

The sewing unit 12 is made up of a sewing portion 12a and a staple receiving portion 12b for clinching the living tissue along with the sewing portion 12a, as shown in Fig.9. The sewing portion 12a, on which are loaded a staple-holding cartridge and a knife blade, has a clinching surface faced by plural staple lines (herein three by three rows of staple lines) and a knife scanning line 15.

The automatic sewing device is inserted into the living body via an attachment termed a serge port. First, the serge port is loaded in a perforation provided in the living body, and the shaft 11 of the automatic sewing device is inserted into the living body via this serge port. This assures hermetic sealing of the perforation formed in the living body.

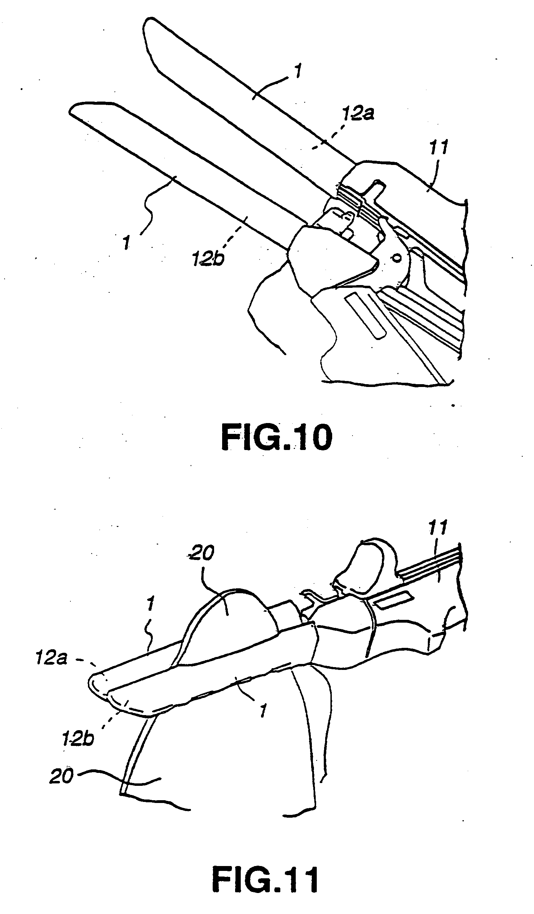

The suture holding member 1 of the present invention is loaded in use on the sewing unit 12 of the automatic sewing device. Fig. 10 shows the state in which the suture holding member 1 is loaded on the sewing unit 12 of the automatic sewing device, with the suture holding member 1 being mounted on each of the sewing portion 12a and the staple receiving portion 12b.

For loading the suture holding member 1 on the sewing unit 12, the suture holding member 1 is introduced into the sewing unit 12 via the opening 2 formed in its proximal end. By setting the inner radius R1 of the suture holding member 1 so as to be smaller than the outer radius R2 of the sewing portion 12a or the outer radius R3 of the staple receiving portion 12b, as shown in Figs. 1 and 9, the suture holding member 1 can be positively held in tight contact with the sewing portion 12a or the staple receiving portion 12b by exploiting elasticity proper to the suture holding member 1.

In this state, the automatic sewing device is inserted via the serge port as described above into the living body to reach the site of lesion. Since the tubular suture holding member 1 having the closed distal end is inserted from the closed end first, there is no risk of offset or twist produced due to contraction.

Since the main body portion of the holding member 2 has the mechanism for preventing detachment from the automatic sewing device, as described above, the main body portion of the holding member 2 can be positively loaded on the sewing portion 12a and to the staple receiving portion 12b thus reliably preventing inadvertent detachment. If the sewing unit 12 is reciprocated in the living body, it is also possible to prevent loading position shifting of the suture holding member 1 with respect to the sewing portion 12a or the staple receiving portion 12b.

After introducing the sewing unit 12 to the site of lesion, the site for resection of a living tissue 20 is clinched between the sewing portion 12 and the staple receiving portion 12b, as shown in Fig.11. This causes the site for resection to be covered by the suture holding member 1.

An operating lever 13a provided on the operating unit 13 of the automatic sewing device is then acted on to drive staples from the staple line 13.

This applies the staples along the site for resection of the living tissue 20 to execute the sewing. At this time, the suture holding member 1 loaded on the sewing unit 12 is applied to the site for resection of the living tissue 20 to effect sewing by the staples.

The suture holding member 1 operates as a reinforcement for sewing. The tissue can be prevented from breakage by sewing the site for resection of the living tissue 20 by the suture holding member 1 to prevent air leakage in the operation of abscission of, for example, great lung cyst.

Then, a knife provided on the sewing portion 12a is scanned along a scanning line 15 to resect the living tissue 20 and the suture holding member 1.

The above process completes the sewing operation. By employing the suture holding member 1 of the present invention, the sewing operation can be executed smoothly, while it is also possible to reinforce the sewing site and to prevent air leakage.

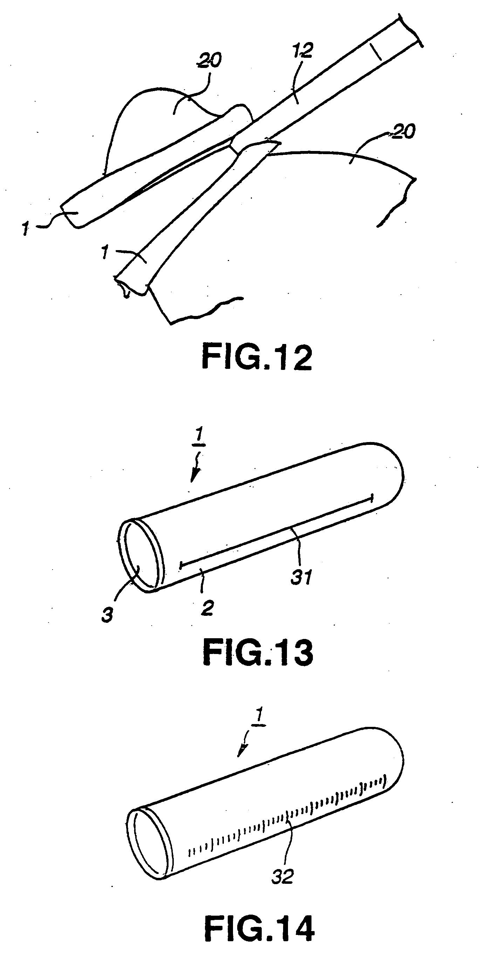

Fig.12 shows the state of end of the sewing operation. In the sewed site of the living tissue 20, the sewing holding member 1 is sewed to assure an extremely stable state of sewing.

The main body portion of the holding member 2 of the sewing holding member 1 according to the present invention is desirably formed of a transparent natural or synthetic high-molecular material. By molding the main body portion of the holding member 2 of a transparent material, the sewed site can be visually checked via the suture holding member 1 when the sewing site of the living tissue is sewed together.

On the outer peripheral surface of the main body portion of the holding member 2 is formed a continuous straight display line 31 from the proximal end to the distal end, as shown in Fig. 13. This display line 31 is provided by forming ribs on the outer peripheral surface of the main body portion of the holding member 2 by printing. By providing the display line 31 in this manner, it can be easily discriminated whether or not the suture holding member 1 has been loaded without distortion on the automatic sewing device.

On the outer peripheral surface of the main body portion of the holding member 2 is formed a graduated line 32 at fixed graduated line intervals, as shown in Fig. 14. By providing the graduated line in this manner, the quantity of the living tissue held by the sewing unit 12 of the automatic sewing device can be comprehended easily to enable the sewing quantity to be checked.

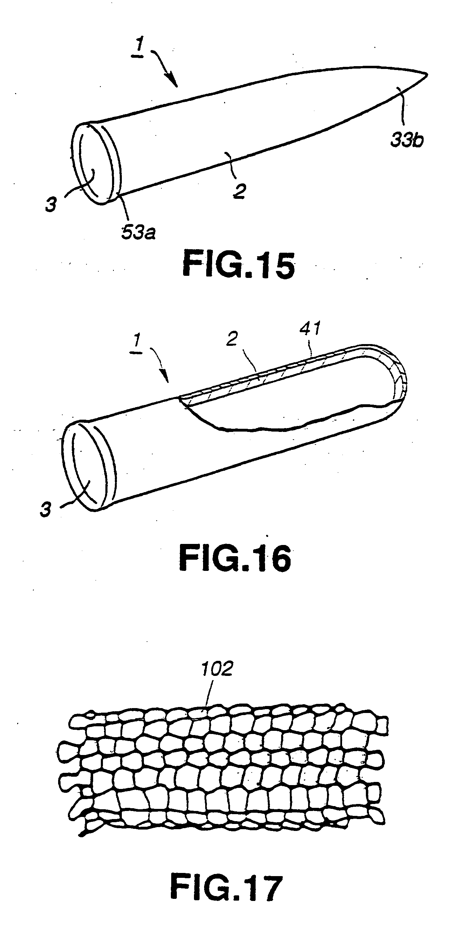

For facilitating loading of the sewing device of the suture holding member 1 on the sewing portion 12a or the staple receiving portion 12b, and for assuring positive loaded state thereof on the sewing portion 12a or the staple receiving portion 12b, the main body portion of the holding member 2 is desirably tapered progressively from the proximal end 33a formed with the opening 3 towards the closed distal end 33b, as shown in Fig.15. By providing a detachment-proofing structure on the main body portion of the holding member 2 in this manner, as described above, the loading state of the sewing device on the sewing portion 12a or the staple receiving portion 12b is assured more reliably, thus preventing possible detachment.

Moreover, the suture holding member 1 according to the present invention includes an insertion smoothing layer 41 formed as a tube integrally 4rom a natural or synthetic high molecular material and which is applied by a hydrophilic polymer on the surface of the main body portion of the holding member 2.

The hydrophilic polymer making up the insertion smoothing layer 41 may be enumerated by methyl polymethacrylate and hydroxyethyl polymethacrylate. More specifically, Vercuflex (trade name) or Hydroplus Coating (trade name) manufactured by Boston Scientific Inc., may be used.

By providing the insertion smoothing layer 41 of a hydrophilic polymer on the surface of the main body portion of the holding member 2, the latter can be smoothly introduced into the living body. That is, when the suture holding member 1 is introduced into the living body, the insertion smoothing layer 41 is combined with the body fluid to improve lubricity to achieve smooth insertion of the suture holding member 1 into the living body.

The main body portion of the holding member 2, formed integrally as a tube, may also be surface-processed to render its surface hydrophilic by immersing the main body portion of the holding member 2 in an alkaline aqueous solution.

The suture holding member 1 according to the present invention is used by being simultaneously mounted on the sewing portion 12a and on the staple receiving portion 12b. That is, the suture holding member 1 is constituted by a pair of the main body portions of the holding members 2.

For facilitated inspection of the loading of the suture holding member 1 on the sewing portion 12a and on the staple receiving portion 12b making up the sewing unit 12 of the automatic sewing device, the paired main body portions of the holding members 2, 2 are of different colors. By using different colors for the paired main body portions of the holding members 2, 2, the sewing portion 12a and on the staple receiving portion 12b can be discriminated from each other by difference in the colors to assure positive insertion of the sewing unit 12 into the living body.

For constituting a suture holding member 101 according to the present invention, a main body portion of the holding members 102, as a tubular knitted product of yarn of natural or synthetic material, is provided, as shown in Fig. 17. The yarn used is prepared by spinning fibers of natural or synthetic material. The knitted article of the main body portion of the holding members 102 is produced by knitting a sole yarn in loops.

Alternatively, the main body portion of the holding members 102 may also be formed by a textile woven in meshes. The textile used here is produced on weaving weft and warp yarns of natural or synthetic material. In particular, the yarns are circular-knitted to produce a tubular fabric.

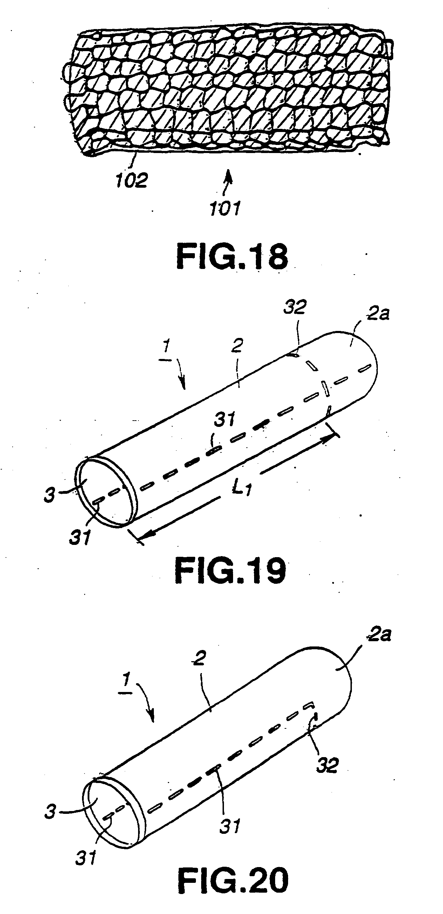

On the surface of the main body portion of the holding members 102, formed as a knitted or woven cloth, an insertion smoothing layer 104 formed of a natural or synthetic high molecular material is provided on the surface of the main body portion of the holding members 102 to smooth the surface of the main body portion of the holding members 102, as shown in Fig. 18. By smoothing the surface of the main body portion of the holding members 102, the suture holding member 101 may be smoothly inserted into the living body

The material of the insertion smoothing layer 104 is also desirably the above-mentioned hydrophilic polymer. Alternatively, the surface of the insertion smoothing layer 104 of the natural or synthetic high molecular material is processed with hydrophilic treatment.

In the suture holding member 1 according to the present invention, in which the main body portion of the holding member is tubular in shape, if the suture holding member 1 is loaded n the automatic sewing device and the site for abscission of a living body is sewed together and subsequently severed, the portion on one end sewed by staples is continuous to the other end sewed with staples to cover the sewing portion 12a and the staple receiving portion 12b of the automatic sewing device. For taking out the severed portion from the living body, the continuous portion of the main body portion of the holding member 2 needs to be severed.

Therefore, in order to facilitate cutting of the continuous portion of the main body portion of the holding member 2 after sewing together and cutting of the site for abscission of the living body, first and second weakened lines 31, 32 are provided on the peripheral surface of the main body portion of the holding member 2, as shown in Fig. 19. Specifically, a pair of first weakened lines 31 are provided for extending from the proximal end towards the distal end along the length of the main body portion of the holding member 2, as shown in Fig. 19. The second weakened line 32 is formed at a distal end of the main body portion of the holding member 2 along the circumference of the main body portion of the holding member 2. The second weakened line 32 is formed at a position corresponding to a length L1 equivalent to the staple line of the automatic sewing device from the proximal end of the main body portion of the holding member 2.

By providing the first and second weakened lines 31, 32, the continuous portion of the main body portion of the holding member 2 can be separated along the first and second weakened lines 31, 32 by sewing and cutting the site of abscission of the living body and by withdrawing the main body portion of the holding member 2. That is, the main body portion of the holding member 2 is cut along the first weakened lines 31, 32, whereby the portions sewed together by staples are separated, and the closed distal end 2a is separated from the main body portion of the holding member 2 by cutting along the second weakened line 32.

The first and second weakened lines 31, 32 are formed by forming perforations or circular or rectangular small holes in succession.

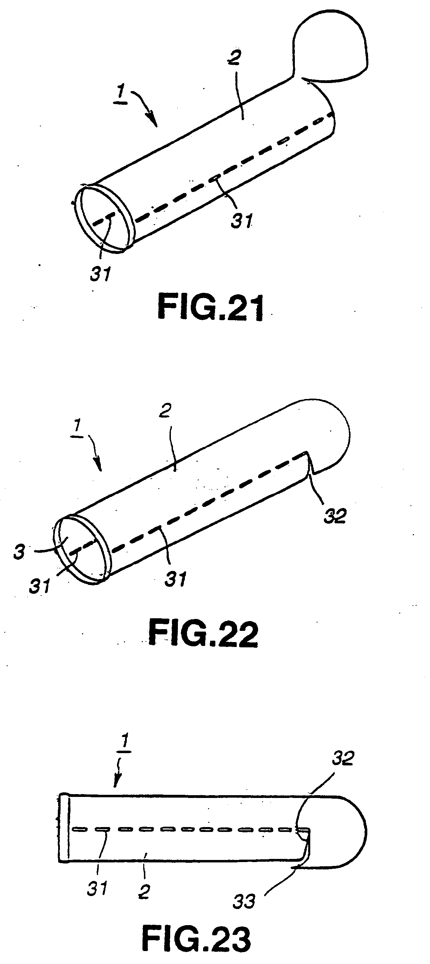

The second weakened line 32 may be formed for extending across the paired first weakened lines 31, 31 along the half circumference of the main body portion of the holding member 2 instead of along its entire circumference, as shown in Fig.20. By forming the second weakened line only in an area extending between the paired first weakened lines 31, 31, the distal end 2a can be formed in continuation to the main body portion of the holding member 2, as shown in Fig.21, to assure facilitated handling after cutting.

Moreover, if the second weakened line 32 is provided across the paired first weakened lines 31, 31 along the half circumference of the main body portion of the holding member 2, a portion of the distal end 2a can be formed in continuation to the main body portion of the holding member 2. Thus, the second weakened line can be formed as a cutting line, as shown in Fig. 22. In this case, the severed portion is formed with a lug 33 protruded towards the main body portion of the holding unit 2. By providing the lug 33, this lug can be held manually and the main body portion of the holding member 2 can be separated easily along the first weakened line 31.

### Industrial Applicability

With the suture holding member according to the present invention, means for inhibiting detachment are provided on the main body portion of the holding member so that the suture holding member can be reliably loaded on the automatic sewing device. In particular, by molding the main body portion of the holding member as a tube closed at its distal end, it is possible to prevent offsetting otherwise caused by contraction in the inserting direction at the time of insertion of the main body portion of the holding member into the automatic sewing device. Moreover, since the loading portion of the medical suture member, molded integrally as a tube closed at its distal end, has its entire surface covered by the suture holding member, the suture holding member can be reliably positioned at the sewing position of the living body.

Also, the medical suture member according to the present invention has its surface treated with hydrophilizing processing, and hence can be inserted extremely smoothly into the living body.

By providing the weakened line in the main body portion of the holding member, the continuous portion of the main body portion of the holding member can be severed easily after suturing and severing the site for abscission of the living body, so that the site for abscission can be removed easily.

## Claims

1. A medical suture holding member (1) loaded in use on an automatic sewing device having a staple line, comprising a main body portion (2)
**characterized in that**
said main body portion (2) of the holding member is molded integrally to a tubular shape from a natural or synthetic high molecular material and having an opening (3) on its proximal end for enabling loading of the main body portion of the holding member on a sewing unit (12) of the automatic sewing device,
wherein said main body portion of the holding member is provided with detachment preventative means for preventing detachment of the suture holding member from said automatic sewing device, and wherein said detachment preventative means is selected from:
- a roughed surface comprised of micro-sized irregularities (5) formed on the inner peripheral surface of the main body portion of the holding member,
- plural ribs (6) formed in the axial direction of the inner peripheral surface of the main body portion of the holding member.
- plural ribs (6) formed in the circumferential direction of the inner peripheral surface of the main body portion of the holding member, and
- plural ribs formed in the axial direction on the inner peripheral surface of the main body portion of the holding member and plural ribs formed in the circumferential direction on the inner peripheral surface of the main body portion of the holding member.

2. The medical suture holding member according to claim 1, wherein said main body portion of the holding member is molded to the shape of a tube having a closed distal end.

3. The medical suture holding member according to claim 1, wherein said high molecular material is a material exhibiting extensibility and flaccidity at ambient temperature.

4. The medical suture holding member according to claim 1 wherein said main body portion of the holding member is molded from a transparent natural or synthetic high molecular material.

5. The medical suture holding member according to claim 1, wherein said main body portion of the holding member is provided with an axially extending straight display line (31).

6. The medical suture holding member according to claim 1, wherein said main body portion of the holding member is provided with a graduated line (32) having a fixed graduation width.

7. The medical suture holding member according to claim 1 wherein said main body portion of the holding member is tapered progressively from its proximal end to its distal end.

8. The medical suture holding member according to claim 1 wherein a hydrophilic polymer is applied to the surface of said main body portion of the holding member.

9. The medical suture holding member according to claim 1, wherein said main body portion of the holding member is processed with hydrophilizing treatment.

10. The medical suture holding member according to claim 1, wherein said high molecular material is a biologically absorptive high molecular material.

11. The medical suture holding member according to claim 1wherein said high molecular material is natural rubber, collagen, gelatine or chitin-chitosan.

12. The medical suture holding member according to claim 1, wherein the synthetic high molecular material is a copolymer containing an aliphatic polyester, a fatty acid anhydride, an aliphatic polycarbonate, polyphosphagen or at least one of these.

13. The medical suture holding member according to claim 1, wherein the synthetic high molecular material is polyglycolic acid, polylactic acid, poly-ε-caprolactone, poly-β-hydroxy alkanoate or a copolymer containing one of these.

14. The medical suture holding member according to claim 1, wherein the synthetic high molecular material is a copolymer containing at least 30 mol% or more of poly-ε-caprolactone.

15. The medical suture holding member according to claim 1, wherein the synthetic high molecular material is a copolymer containing at least 30 mol% or more of poly-β-hydroxy alkanoate.

16. The medical suture holding member according to claim 1 wherein the synthetic high molecular material is a mixture of a natural high molecular material or a synthetic high molecular material.

17. The medical suture holding member according to claim 1,
**characterized in that** it comprises a pair of main body portions of holding members, wherein said pair of main body portions of holding members being of different colors.

18. The medical suture holding member according to claim 1, wherein said main body portion of a holding member has a weakened line.

19. The medical suture holding member according to claim 18, wherein said weakened line includes a first weakened line formed for extending from and opening end side towards the distal end of said main body portion of a holding member and a second weakened line formed towards the distal end of the main body portion of the holding member for extending along the circumference of said main body portion of the holding member.

20. The medical suture holding member according to claim 19, wherein the first, weakened line is comprised of a pair of weakened lines formed for extending from an opening end side towards the distal end of said main body portion of a holding member and wherein the second weakened line is formed for approximately a half circumference of said main body portion of a holding member between said pair of the first weakened lines.

21. The medical suture holding member according to claim 20, wherein the second weakened line is formed as a cutting line and a holding lug is provided on a sectional surface of the distal end of the main body portion of a holding member.

## Patentansprüche

1. Organ (1) zum Halten eines medizinischen Nähfadens, das im Gebrauch in eine automatische Nähvorrichtung, die eine Klammerlinie besitzt, geladen ist und einen Hauptkörperabschnitt (2) aufweist,
**dadurch gekennzeichnet, dass**
der Hauptkörperabschnitt (2) des Halteorgans einteilig in eine Rohrform aus einem natürlichen oder synthetischen hochmolekularen Material gegossen ist und an seinem proximalen Ende eine Öffnung (3) besitzt, um das Laden des Hauptkörperabschnitts des Halteorgans in eine Näheinheit (12) der automatischen Nähvorrichtung zu ermöglichen,
wobei der Hauptkörperabschnitt des Halteorgans mit Ablöseverhinderungsmitteln versehen ist, um eine Ablösung des Nähfadenhalteorgans von der automatischen Nähvorrichtung zu verhindern, und wobei die Ablöseverhinderungsmittel ausgewählt sind aus:
- einer aufgerauten Oberfläche, die Irregularitäten (5) in Mikrogröße aufweist, die an der inneren Umfangsoberfläche des Hauptkörperabschnitts des Halteorgans ausgebildet sind,
- mehreren Rippen (6), die in axialer Richtung der inneren Umfangsoberfläche des Hauptkörperabschnitts des Halteorgans ausgebildet sind,
- mehreren Rippen (6), die in Umfangsrichtung der inneren Umfangsoberfläche des Hauptkörperabschnitts des Halteorgans ausgebildet sind, und
- mehreren Rippen, die in axialer Richtung an der inneren Umfangsoberfläche des Hauptkörperabschnitts des Halteorgans ausgebildet sind, und mehreren Rippen, die in Umfangsrichtung an der inneren Umfangsoberfläche des Hauptkörperabschnitts des Halteorgans ausgebildet sind.

2. Organ zum Halten eines medizinischen Nähfadens nach Anspruch 1, bei dem der Hauptkörperabschnitt des Halteorgans in die Form eines Rohrs mit einem geschlossenen distalen Ende gegossen ist.

3. Organ zum Halten eines medizinischen Nähfadens nach Anspruch 1, bei dem das hochmolekulare Material ein Material ist, das bei Umgebungstemperatur eine Streckbarkeit und eine Schlaffheit zeigt.

4. Organ zum Halten eines medizinischen Nähfadens nach Anspruch 1, bei dem der Hauptkörperabschnitt des Halteorgans aus einem transparenten, natürlichen oder synthetischen hochmolekularen Material gegossen ist.

5. Organ zum Halten eines medizinischen Nähfadens nach Anspruch 1, bei dem der Hauptkörperabschnitt des Halteorgans mit einer axial verlaufenden geraden Anzeigelinie (31) versehen ist.

6. Organ zum Halten eines medizinischen Nähfadens nach Anspruch 1, bei dem der Hauptkörperabschnitt des Halteorgans mit einer Skalenlinie (32) mit fester Skalenteilung versehen ist.

7. Organ zum Halten eines medizinischen Nähfadens nach Anspruch 1, bei dem der Hauptkörperabschnitt des Halteorgans von seinem proximalen Ende zu seinem distalen Ende progressiv konisch zuläuft.

8. Organ zum Halten eines medizinischen Nähfadens nach Anspruch 1, bei dem auf die Oberfläche des Hauptkörperabschnitts des Halteorgans ein hydrophiles Polymer aufgebracht ist.

9. Organ zum Halten eines medizinischen Nähfadens nach Anspruch 1, bei dem der Hauptkörperabschnitt des Halteorgans mit einer Hydrophiliebehandlung bearbeitet wird.

10. Organ zum Halten eines medizinischen Nähfadens nach Anspruch 1, bei dem das hochmolekulare Material ein biologisch absorbierendes hochmolekulares Material ist.

11. Organ zum Halten eines medizinischen Nähfadens nach Anspruch 1, bei dem das hochmolekulare Material natürlicher Kautschuk, Kollagen, Gelatine oder Chitin-Chitosan ist.

12. Organ zum Halten eines medizinischen Nähfadens nach Anspruch 1, bei dem das synthetische hochmolekulare Material ein Copolymer ist, das einen aliphatischen Polyester, ein Fettsäure-Anhydrid, ein aliphatisches Polycarbonat, Polyphosgen (Polyphosphagen) oder zumindest eines hiervon enthält.

13. Organ zum Halten eines medizinischen Nähfadens nach Anspruch 1, bei dem das synthetische hochmolekulare Material Polyglycolsäure, PolyMilchsäure, Poly-ε-Caprolacton, Poly-β-Hydroxy-Alkanoat oder ein Copolymer, das eines hiervon enthält, ist.

14. Organ zum Halten eines medizinischen Nähfadens nach Anspruch 1, bei dem das synthetische hochmolekulare Material ein Copolymer ist, das wenigstens 30 Mol-% oder mehr Poly-ε-Caprolacton enthält.

15. Organ zum Halten eines medizinischen Nähfadens nach Anspruch 1, bei dem das synthetische hochmolekulare Material ein Copolymer ist, das wenigstens 30 Mol-% oder mehr Poly-β-Hydroxy-Alkanoat enthält.

16. Organ zum Halten eines medizinischen Nähfadens nach Anspruch 1, bei dem das synthetische hochmolekulare Material ein Gemisch aus einem natürlichen hochmolekularen Material und aus einem synthetischen hochmolekularen Material ist.

17. Organ zum Halten eines medizinischen Nähfadens nach Anspruch 1, **dadurch gekennzeichnet, dass** es ein Paar Hauptkörperabschnitte von Halteorganen aufweist, wobei die beiden Hauptkörperabschnitte von Halteorganen unterschiedliche Farben haben.

18. Organ zum Halten eines medizinischen Nähfadens nach Anspruch 1, bei dem der Hauptkörperabschnitt eines Halteorgans eine geschwächte Linie besitzt.

19. Organ zum Halten eines medizinischen Nähfadens nach Anspruch 18, bei dem die geschwächte Linie eine erste geschwächte Linie, die so ausgebildet ist, dass sie sich von einer Öffnungsstirnseite zu dem distalen Ende des Hauptkörperabschnitts eines Halteorgans erstreckt, und eine zweite geschwächte Linie, die in Richtung zu dem distalen Ende des Hauptkörperabschnitts des Halteorgans ausgebildet ist, um sich längs des Umfangs des Hauptkörperabschnitts des Halteorgans zu erstrecken, enthält.

20. Organ zum Halten eines medizinischen Nähfadens nach Anspruch 19, bei dem die erste geschwächte Linie ein Paar geschwächter Linien enthält, die so ausgebildet sind, dass sie sich von einer Öffnungsstirnseite zu dem distalen Ende des Hauptkörperabschnitts eines Halteorgans erstrecken, und bei dem die zweite geschwächte Linie so ausgebildet ist, dass sie sich etwa über den halben Umfang des Hauptkörperabschnitts des Halteorgans zwischen dem Paar erster geschwächter Linien erstreckt.

21. Organ zum Halten eines medizinischen Nähfadens nach Anspruch 20, bei dem die zweite geschwächte Linie als eine Schneidlinie ausgebildet ist und an einer Querschnittsfläche des distalen Endes des Hauptkörperabschnitts eines Halteorgans ein Halteansatz vorgesehen ist.

## Revendications

1. Elément de maintien de suture de type médical (1) chargé pendant l'utilisation sur un dispositif de suture automatique comportant une ligne d'agrafes, comprenant une partie de corps principal (2), **caractérisé en ce que** ladite partie de corps principal (2) de l'élément de maintien est intégralement moulée sous une forme tubulaire à partir d'un matériau naturel ou synthétique à haut poids moléculaire et comporte une ouverture (3) sur son extrémité proximale permettant de charger la partie de corps principal de l'élément de maintien sur une unité de suture (12) du dispositif de suture automatique,
élément dans lequel ladite partie de corps principal est pourvue de moyens de prévention de détachement pour empêcher le détachement de l'élément de maintien de suture dudit dispositif de suture automatique, et dans lequel lesdits moyens de prévention de détachement sont sélectionnés parmi :
- une surface rugueuse composée d'irrégularités de taille microscopique (5) formées sur la surface périphérique interne de la partie de corps principal de l'élément de maintien,
- plusieurs nervures (6) formées dans le sens axial de la surface périphérique interne de la partie de corps principal de l'élément de maintien,
- plusieurs nervures (6) formées dans le sens circonférentiel de la surface périphérique interne de la partie de corps principal de l'élément de maintien, et
- plusieurs nervures formées dans le sens axial sur la surface périphérique interne de la partie de corps principal de l'élément de maintien et plusieurs nervures formées dans le sens circonférentiel sur la surface périphérique interne de la partie de corps principal de l'élément de maintien.

2. Elément de maintien de suture de type médical selon la revendication 1, dans lequel ladite partie de corps principal est moulée sous la forme d'un tube ayant une extrémité distale fermée.

3. Elément de maintien de suture de type médical selon la revendication 1, dans lequel ledit matériau à haut poids moléculaire est un matériau pourvu d'extensibilité et de flaccidité à la température ambiante.

4. Elément de maintien de suture de type médical selon la revendication 1, dans lequel ladite partie de corps principal de l'élément de maintien est moulée à partir d'un matériau transparent naturel ou synthétique à haut poids moléculaire.

5. Elément de maintien de suture de type médical selon la revendication 1, dans lequel ladite partie de corps principal de l'élément de maintien est pourvue d'une ligne d'affichage droite s'étendant axialement (31).

6. Elément de maintien de suture de type médical selon la revendication 1, dans lequel ladite partie de corps principal de l'élément de maintien est pourvue d'une ligne graduée (32) ayant une largeur de graduation fixe.

7. Elément de maintien de suture de type médical selon la revendication 1, dans lequel ladite partie de corps principal de l'élément de maintien est progressivement réduite de son extrémité proximale à son extrémité distale.

8. Elément de maintien de suture de type médical selon la revendication 1, dans lequel un polymère hydrophile est appliqué à la surface de ladite partie de corps principal de l'élément de maintien.

9. Elément de maintien de suture de type médical selon la revendication 1, dans lequel ladite partie dé corps principal de l'élément de maintien fait l'objet d'un traitement d'hydrophilisation.

10. Elément de maintien de suture de type médical selon la revendication 1, dans lequel ledit matériau à haut poids moléculaire est un matériau à haut poids moléculaire biologiquement absorbant.

11. Elément de maintien de suture de type médical selon la revendication 1, dans lequel ledit matériau à haut poids moléculaire est de la gomme naturelle, du collagène, de la gélatine ou du chitin-chitosan.

12. Elément de maintien de suture de type médical selon la revendication 1, dans lequel le matériau synthétique à haut poids moléculaire est un copolymère contenant un polyester aliphatique, un anhydride d'acide gras, un polycarbonate aliphatique, un polyphosphagène ou au moins l'un d'entre eux.

13. Elément de maintien de suture de type médical selon la revendication 1, dans lequel le matériau synthétique à haut poids moléculaire est l'acide polyglycolique, l'acide polylactique, la poly-ε-caprolactone, le poly-β-hydroxyalcanoate ou un copolymère contenant l'un d'entre eux.

14. Elément de maintien de suture de type médical selon la revendication 1, dans lequel le matériau synthétique à haut poids moléculaire est un copolymère contenant au moins 30 % en moles ou plus de poly-ε-caprolactone.

15. Elément de maintien de suture de type médical selon la revendication 1, dans lequel le matériau synthétique à haut poids moléculaire est un copolymère contenant au moins 30 % en moles ou plus de poly-β-hydroxyalcanoate.

16. Elément de maintien de suture de type médical selon la revendication 1, dans lequel le matériau synthétique à haut poids moléculaire est un mélange d'un matériau naturel à haut poids moléculaire ou d'un matériau synthétique à haut poids moléculaire.

17. Elément de maintien de suture de type médical selon la revendication 1, **caractérisé en ce qu'**il comprend deux parties de corps principal d'éléments de maintien, lesdites deux parties de corps principal d'éléments de maintien étant de couleurs différentes.

18. Elément de maintien de suture de type médical selon la revendication 1, dans lequel ladite partie de corps principal de l'élément de maintien comprend une ligne fragilisée.

19. Elément de maintien de suture de type médical selon la revendication 18, dans lequel ladite ligne fragilisée comprend une première ligne fragilisée formée pour s'étendre depuis un côté d'extrémité d'ouverture vers l'extrémité distale de ladite partie de corps principal d'un élément de maintien et une seconde ligne fragilisée formée vers l'extrémité distale de la partie de corps principal de l'élément de maintien pour s'étendre le long de la circonférence de ladite partie de corps principal de l'élément de maintien.

20. Elément de maintien de suture de type médical selon la revendication 19, dans lequel la première ligne fragilisée est constituée d'une paire de lignes fragilisées formées pour s'étendre depuis un côté d'extrémité d'ouverture vers l'extrémité distale de ladite partie de corps principal d'un élément de maintien et dans lequel la seconde ligne fragilisée est formée sur approximativement une demi-circonférence de ladite partie de corps principal d'un élément de maintien entre ladite paire de premières lignes fragilisées.

21. Elément de maintien de suture de type médical selon la revendication 20, dans lequel la seconde ligne fragilisée est formée en tant que ligne de coupe et une patte de maintien est prévue sur une surface de section de l'extrémité distale de la partie de corps principal de l'élément de maintien.
